# EUROPÄISCHE PATENTSCHRIFT

(11) **EP 0 595 438 B1**
(45) Veröffentlichungstag und Bekanntmachung des Hinweises auf die Patenterteilung: **01.12.1999**
(21) Anmeldenummer: 93250209.9
(22) Anmeldetag: 14.07.1993
(51) Int. Cl.: B07C 5/14, G01N 33/46, G01N 21/89

(54) **Anlage zur Bestimmung der Holzgüte von Schnitthölzern**
Plant for determining the quality of sawwood
Installation de détermination de la qualité de bois de sciage

(30) Priorität: 15.07.1992 DE 4223787
(43) Veröffentlichungstag der Anmeldung: 04.05.1994
(73) Patentinhaber: Dimter GmbH Maschinenfabrik, D-89257 Illertissen (DE)
(72) Erfinder: Fröhlich, Adolf, Dipl.-Ing., D-89281 Altenstadt (DE)
(74) Vertreter: Maikowski, Michael, Dipl.-Ing. Dr.

(56) Entgegenhaltungen:
- EP-A- 0 234 492
- WO-A-90/11488
- DE-A- 4 033 485
- US-A- 4 207 472
- US-A- 4 221 974

## Beschreibung

Die Erfindung bezieht sich auf eine Anlage zur Bestimmung der Holzgüte nach dem Oberbegriff des Anspruchs 1.

Ein derartiger Stand der Technik ist aus der WO-A-9011488 bekannt.

Die Bestimmung der Holzgüte ist eine Voraussetzung zur Sortierung von Schnitthölzern, insbesondere von Schnitthölzern, die für tragende Bauteile bestimmt sind. Diese Schnitthölzer werden nach der Tragfähigkeit sortiert.

Sortierkriterien zur Sortierung von Schnitthölzern nach der Tragfähigkeit sind beispielsweise in der DIN 4074 und in der ECE-Norm prEN 338 festgelegt.

Es sind Sortiermaschinen bekannt, in denen das Schnittholz bei der Sortierung beansprucht wird. Bei dieser Beanspruchung wird die Durchbiegung gemessen. Die Maschine ermittelt hieraus den E-Modul.

Gewisse Holzmerkmale, insbesondere Oberflächenfehler und Rohdichte werden dabei nicht berücksichtigt. Eine Maschine, die die Kriterien der Sortierklasse MS 17 nach DIN 4074 und/oder der ECE-Norm prEN 338 erfüllt, steht zur Zeit nicht zur Verfügung.

Beim belastungfreien oder beanspruchungsfreien Sortieren wird das Schnittholz auf Holzfehler untersucht, die an der Oberfläche des Holzes sichtbar sind. Die Sortiermerkmale, die durch eine Inaugenscheinnahme durch das Bedienungspersonal festgestellt werden, sind in der DIN 4074 unter Punkt 4 definiert. Nach diesen Merkmalen werden diese Hölzer in Sortierklassen eingeteilt, die der Tabelle 2 der DIN 4074 zu entnehmen sind.

Die Qualität einer derartigen Sortierung hängt von der Erfahrung des Personals ab, welches die Inaugenscheinnahme vornimmt. Darüberhinaus ist diese Sortierung grobstufig, wodurch eine optimale Nutzung des zur Verfügung stehenden Schnittholzes verhindert wird.

Der Erfindung liegt die Aufgabe zugrunde, eine Anlage zur Verfügung zu stellen, mit der mit einfachsten Mitteln maschinell eine präzise feinstufige Sortierung möglich ist, so daß das Schnittholz besser ausgenutzt werden kann.

Erfindungsgemäß wird diese Aufgabe durch die technische Lehre des Inhalts des Anspruchs 1 gelöst.

Mit Vorteil erfaßt der Scanner die vier Längsseiten der Schnitthölzer und tastet die auf den Oberflächen sichtbaren Fehler ab. Diese Fehlersignale werden in einen Rechner eingegeben. In den Rechner wurde ein Fehlermuster eingespeichert und dieses Fehlermuster kann beispielsweise die Sortiermerkmale umfassen, die in der DIN 4074 Teil 1 angegeben sind. Es besteht die Möglichkeit dabei, die an das Holz zu stellenden Kriterien entsprechend der DIN 4074 auszuwählen. Der Fachmann hat mit Vorteil die Möglichkeit, zusätzliche oder andere Sortierkriterien aufzustellen und einzugeben, wie die der ECE-Norm prEN 338.

Im Rechner werden die vom Scanner abgetasteten Fehlersignale mit den eingespeicherten Mustern verglichen. Dieser Vergleich ermöglicht eine bewertende Vorklassierung.

Gleichzeitig werden im Scanner bei der Abtastung Länge, Breite und Höhe des Schnittholzes gemessen, und aus diesen Größen wird im Rechner das Volumen des abgetasteten Schnittholzes ermittelt. Desweiteren wird in einer Wiegestation das Gewicht des zu bewertenden Schnittholzes bestimmt. Da das Volumen bekannt ist, kann nunmehr die Rohdichte des Schnittholzes ermittelt werden.

Da bekanntlich bei der Ermittlung der Rohdichte des Schnittholzes die Holzfeuchtigkeit mit angegeben wird, ist, falls die Holzfeuchtigkeit nicht bekannt ist, in der Anlage eine Einrichtung zur Messung der Holzfeuchtigkeit vorzusehen.

Wenn dem Durchschnittsfachmann die Auswertung der Fehlererkennung vorliegt und zusätzlich die Rohdichte des Schnittholzes in Abhängigkeit von der Feuchtigkeit bekannt ist, kann dieser eine feinstufigere Sortierung vornehmen, da die Rohdichte einen Parameter darstellt, mit der die Zug- und Druckfestigkeit der zu bestimmenden Holzarten ermittelt werden kann.

Außerdem kann aus der Abtastung empirisch ein Korrekturfaktor für die Rohdichte ermittelt und mit der korrigierten Rohdichte aus bekannten Tabellenwerken ein Festigkeitswert für das Holz bestimmt werden.

Die erfindungsgemäße Anlage ermöglicht mit Vorteil eine Berechnung der Festigkeit innerhalb der 95% Fraktile, d.h. die effektive Festigkeit ist zu 95% größer als die berechneten, so daß eine ausreichende Sicherheit gegeben ist. Die Bestimmung der Fraktile kann gemäß ECE-Norm prEN 384 5.3 durchgeführt werden.

Da es zur Optimierung einer feinstufigen Sortierung nützlich ist, den mittleren E-Modul des Holzes in Längsrichtung, das heißt in Faserrichtung zu kennen, ist in vorteilhafter Weise in der Anlage eine Station mit einem, auf die Schnitthölzer einwirkenden mechanischen Impulsgeber und einem mit dem Rechner verbundenen Meßwandler zur Aufnahme der in den Schnitthölzern durch den Impulsgeber angeregten Schwingungen vorgesehen. Diese Station ermöglicht eine dynamische Bestimmung des E-Moduls durch Impulserregung und Frequenzanalyse. Das Schnittholz wird stirnseitig durch einen leichten mechanischen Impuls zu einer Longitutinal-Grundschwingung angeregt, die vom Meßwandler aufgenommen wird. Es wird der Nulldurchgang des vom Meßwandler übertragenen Signals festgestellt. Aus der Frequenz, bei der dieser Nulldurchgang erfolgt, aus der festgestellten Rohdichte und aus der ermittelten Länge der Schnitthölzer wird der Elastizitätsmodul in Längsrichtung ermittelt. Hierzu wird die bekannte Beziehung zwischen der Frequenz, den Eigenschwingungen eines stabförmigen Körpers und dessen Länge, E-Modul und Dichte angewendet.

In der DIN 4074, Teil 3 wird im Abschnitt 3.4 "Durchführung" dargelegt, daß der schwächste Querschnitt zuverlässig erkannt werden muß. Dieser bestimmt die Güteklasse des geprüften Holzes. Da sowohl Dicke und gegebenenfalls der E-Modul als Mittelwerte erfaßt werden können, ist der Scanner wesentlich für die Erfassung des schwächsten Querschnitts. Der Scanner erfaßt die am Holz vorhandenen Äste, insbesondere deren Lage, Größe, Art und die Astkonzentration.

Mit Vorteil werden im Scanner CCD-Zeilenkameras verwendet. Diese Kameras weisen CCD-Sensoren auf, die im Durchlauf die Oberflächen des Schnittholzes im Pixel mit unterschiedlichen Graustufen einteilen. Die Graustufenwerte und Verteilungen, die von der Kamera abgegeben werden, werden anschließend im Rechner analysiert und mit vorher eingegebenen Fehlerkriterien verglichen. Dieser Vergleich ermöglicht eine hochpräzise kritische Bewertung der Oberflächenabtastung, die wird durch die gleichzeitig ermittelte Rohdichte ergänzt. Mit besonderem Vorteil wird in der Anlage der Scanner nicht nur zur Abtastung der Oberflächen verwendet, sondern auch als Meßinstrument, das an der Ermittlung der für die Bewertung der Holzgüte wichtigen Rohdichte beim Durchlauf beteiligt ist.

Mit besonderem Vorteil weist die Anlage einen, einen Längsförderer beschickenden Querförderer auf, in dessen Förderbahn die Wiegestation, die Station mit dem mechanischen Impulsgeber und der Meßwertwandler und gegebenenfalls die Feuchtigkeitsmeßstation angeordnet sind. Der Scanner ist in der Förderbahn des Längsförderers angeordnet. Bei diesem Aufbau wird eine optimale Ausnutzung des Raumes ermöglicht. Der Querförderer kann über eine Vereinzelungsstation von einem Stapel beschickt werden.

Mit besonderem Vorteil sind dem Scanner Sortier- und/oder eine Markierstation nachgeschaltet. In diesen Stationen werden Sortier- oder Markiermaßnahmen entsprechend der vom Rechner abgegebenen Bewertungen vorgenommen.

Der Scanner ermöglicht in vorteilhafter Weise zwei Hölzer, insbesondere Lamellen, gleicher Güteklasse noch zusätzlich nach ästhetischen Forderungen (optischer Eindrücke bei Verwendung als Sichtlamellen) zu sortieren.

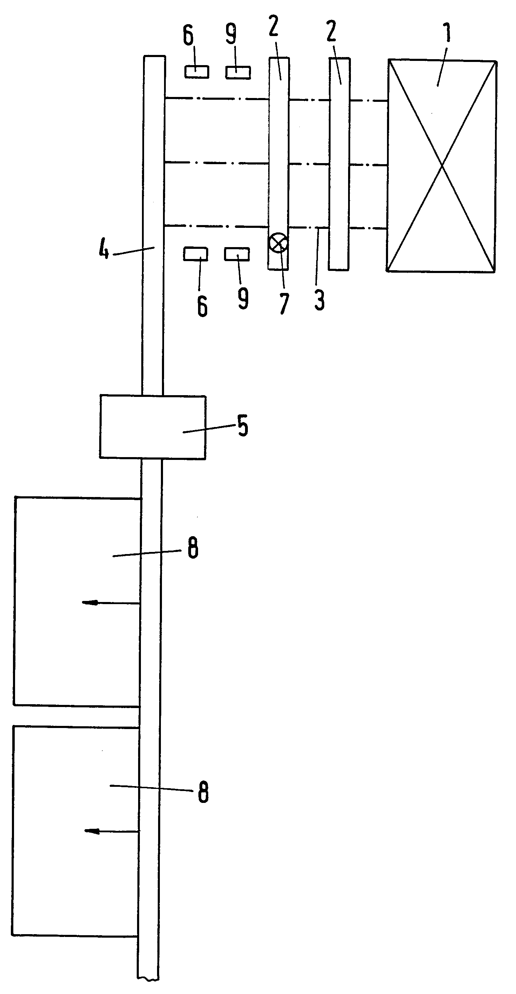

Ein Ausführungsbeispiel soll unter Bezugnahme auf die Zeichnung, die eine schematische Draufsicht auf eine Anlage darstellt, erläutert werden.

Aus dem Holzstapel 1 werden Schnitthölzer 2 vereinzelt und auf einen Querförderer 3 abgegeben. Dieser Querförderer 3 beschickt einen Längsförderer 4.

In der Förderbahn des Querförderers 3 sind eine Wiegestation 6 und gegebenenfalls eine Meßstation 7 für die Holzfeuchtigkeit angeordnet. Das in der Wiegestation 6 gewogene Schnittholz 2 wird vom Längsförderer 4 durch den Scanner 5 hindurchgeführt.

Der Scanner 5 und die Wiegestation 6 und gegebenenfalls die Meßstation 7 sind mit einem nicht dargestellten Rechner zu einer Funktionseinheit zusammengefaßt. In dieser Funktionseinheit werden die ermittelten Parameter ausgewertet. In den Klassierstationen 8 werden dann die Schnitthölzer 2 nach im Rechner erhaltenen Klassiermerkmalen sortiert. Es besteht auch die Möglichkeit, die Schnitthölzer 2 in einer Station zu kennzeichnen.

## Patentansprüche

1. Anlage zur Bestimmung der durch Sortierkriterien vorgegbenen Holzgüte von Schnitthölzern.
**gekennzeichnet durch**
einen Scanner (5) mit vierseitiger Kameraerfassung zur Abtastung der vier Längsseiten der Schnitthölzer (2) auf an deren Oberflächen sichtbare Fehler und zur Messung der Längen, Breiten und Höhen der Schnitthölzer (2) und eine Wiegestation (6) für die Schnitthölzer, deren Ausgänge zum Vergleich der vom Scanner (5) abgetasteten Oberflächenfehler mit eingespeicherten Fehlermustern und zur Ermittlung der Rohdichte der Schnitthölzer (2) mit einem Rechner verbunden sind.

2. Anlage nach Anspruch 1, **gekennzeichnet durch** eine Feuchtigkeitsmeßstation (7) für die Schnitthölzer.

3. Anlage nach Anspruch 1 oder 2, **gekennzeichnet durch** eine Station (9) mit einem auf die Schnitthölzer (2) einwirkenden mechanischen Impulsgeber und einem mit dem Rechner verbundenen Meßwandler zur Aufnahme der in den Schnitthölzern (2) durch den Impulsgeber angeregten Schwingungen.

4. Anlage nach Anspruch 3, **dadurch gekennzeichnet,** daß in der Station (9) der mechanische Impulsgeber auf die Stirnseite der Schnitthölzer einwirkend angeordnet ist.

5. Anlage nach mindestens einem der Ansprüche 1-4, **dadurch gekennzeichnet,** daß die Kameras des Scanners (5) CCD-Zeilenkameras sind.

6. Anlage nach mindestens einem der Ansprüche 1-5, **dadurch gekennzeichnet,** daß der Scanner (5), die Wiegestation (6), die Station (9) mit dem mechanischen Impulsgeber und Meßwertwandler und gegebenenfalls die Feuchtigkeitsmeßstation (7) an oder in einer Förderstrecke für die Schnitthölzer (2) angeordnet sind.

7. Anlage nach Anspruch 6, **dadurch gekennzeichnet,** daß die Förderstrecke einen, einen Längsförderer (4) beschickenden Querförderer (3) aufweist, in dessen Förderbahn die Wiegestation (6), die Station (9) mit dem mechanischen Impulsgeber und Meßwertwandler und gegebenenfalls die Feuchtigkeitsmeßstation (7) angeordnet sind, und daß der Scanner (5) in der Förderbahn des Längsförderers (4) angeordnet ist.

8. Anlage nach Anspruch 4 oder 5, **dadurch gekennzeichnet,** daß dem Scanner (5) Klassierstationen (8) und/oder eine Markierstation nachgeschaltet sind.

## Claims

1. Apparatus for determining the wood quality of cut timber derived from sorting criteria,
characterised by
a scanner (5) with four sided camera facilities for sampling the four longitudinal sides of the cut timber (2) for visible faults on the surface and for measuring the length, width and thickness of the cut timber (2) and a weighing station (6) for the cut timber, the output of which is connected with a computer for comparison of the surface faults sensed by the scanner (5) with stored fault patterns and for outputting the raw density of the cut timber (2).

2. Apparatus according to Claim 1, characterised by a moisture measurement station (7) for the cut timber.

3. Apparatus according to Claim 1 or 2, characterised by a station (9) with a mechanical pulse generator acting on the cut timber (2) and a transmitter connected with the computer for receiving the oscillations imparted to the cut timber (2) by the pulse generator.

4. Apparatus according to Claim 3, characterised in that the mechanical pulse generator is arranged to act on the end face of the cut timber in the station (9).

5. Apparatus according to at least one of claims 1 - 4, characterised in that the cameras of the scanner (5) are CCD-line cameras.

6. Apparatus according to at least one of the claims 1 - 5, characterised in that the scanner (5), the weighing station (6), the station (9) with the mechanical pulse generator and measured value transmitter and possibly the moisture measurement station (7) are arranged on or in a conveyor path for the cut timber.

7. Apparatus according to Claim 6, characterised in that the conveying path has a transverse conveyor (3) loading a longitudinal conveyor (4) and the weighing station (6), the station (9) with the mechanical pulse generator and measured value transmitter and possibly the moisture measurement station (7) are arranged in the transverse conveyor path, and that the scanner (5) is arranged in the conveyor path of the longitudinal conveyor (4).

8. Apparatus according to Claim 4 or 5, characterised in that the scanner (5), classification stations (8) and/or a marking station are arranged one after the other.

## Revendications

1. Installation pour le contrôle de la qualité prédéfinie du bois de sciage par des critères de tri,
**caractérisée par**
un scanner (5) avec saisie sur quatre côtés par caméra pour détecter sur les quatre côtés longitudinaux des bois de sciage (2) les défauts visibles sur leurs surfaces et pour mesurer les longueurs, largeurs et hauteurs des bois de sciage (2) et un poste de pesage (6) pour les bois de sciage, scanner dont les sorties sont raccordées à un ordinateur en vue de la comparaison des défauts de surface détectés par le scanner (5) avec des échantillons de défauts mis en mémoire et servant à déterminer la masse volumique apparente des bois de sciage (2).

2. Installation selon la revendication 1, **caractérisée par** un poste de mesure de l'humidité (7) pour les bois de sciage.

3. Installation selon la revendication 1 ou la revendication 2, **caracterisée par** un poste (9) muni d'un générateur d'impulsions mécanique agissant sur les bois de sciage (2) et un convertisseur de mesure raccordé à l'ordinateur pour enregistrer les vibrations provoquées par le générateur d'impulsions dans les bois de sciage.

4. Installation selon la revendication 3, **caractérisée par** le fait que dans le poste (9) le générateur d'impulsions mécanique est agencé de façon à pouvoir agir sur le côté frontal des bois de sciage.

5. Installation selon l'une des revendications 1-4, **caractérisée par** le fait que les caméras du scanner (5) sont des caméras à cellules CCD.

6. Installation selon au moins l'une des revendications 1 - 5, **caractérisée par** le fait que le scanner (5), le poste de pesage le poste (9) avec le générateur d'impulsions mécanique et le convertisseur de mesure et éventuellement le poste de mesure de l'humidité (7) sont agencés sur ou dans une voie d'acheminement ou de manutention pour les bois de sciage (2).

7. Installation selon la revendication 6, **caractérisée par** le fait que la voie d'acheminement ou de manutention comporte un dispositif transporteur ou convoyeur transversal (3) alimentant un dispositif transporteur ou convoyeur longitudinal (4), dans la voie d'acheminement ou de manutention desquels sont implantés le poste de pesage (6),, le poste (9) avec le générateur d'impulsions mécanique et le convertisseur de mesure et éventuellement le poste de mesure de l'humidité (7) et par le fait que le scanner (5) est disposé dans la voie d'acheminement ou de manutention du dispositif transporteur ou convoyeur (4).

8. Installation selon la revendication 4 ou la revendication 5, **caractérisée par** le fait qu'en aval du scanner (5) sont implantés les postes de tri (8) et/ou un poste de marquage.
